(19)

Europäisches
Patentamt
European
Patent Office
Office européen
des brevets

(11) **EP 1 980 245 A1**

(12) **EUROPEAN PATENT APPLICATION**

(43) Date of publication:
**15.10.2008 Bulletin 2008/42**

(51) Int Cl.:
*A61K 9/24* (2006.01)          *A61K 31/165* (2006.01)
*A61K 31/135* (2006.01)

(21) Application number: **07290444.4**

(22) Date of filing: **11.04.2007**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(71) Applicants:
• **CEPHALON FRANCE**
**94700 Maisons-Alfort (FR)**
• **CEPHALON, INC.**
**Frazer, PA 19355 (US)**

(72) Inventors:
• **Dulieu, Claire**
**94370 Sucy en Brie (FR)**

• **Durfee, Steve**
**Sandy, UT 84092 (US)**
• **Holl, Richard**
**Park City, UT 84098 (US)**
• **Nguyên, Thanh-Tâm**
**94450 Limeil-Brevannes (FR)**
• **Phadungpojna, Sasima**
**North Salt Lake, Utah 84054 (US)**

(74) Representative: **Colombet, Alain André et al**
**Cabinet Lavoix**
**2, place d'Estienne d'Orves**
**75441 Paris Cedex 03 (FR)**

(54) **Bilayer lyophilized pharmaceutical compositions and methods of making and using same**

(57)     The present invention relates to a bilayer dosage form comprising:
- an upper layer (a) comprising a lyophilized dosage form of active pharmaceutical ingredient(s) (API(s)).

- a base line layer (b) formulated to adhere to the oral mucosa and intended for delayed, sustained or extended release of API(s) and/or excipient(s).

EP 1 980 245 A1

**Description**

BACKGROUND OF THE INVENTION

Oral administration is one of the most common methods of administering pharmaceutical compositions. The pharmaceutical compositions are generally formulated as a solid dosage form (such as a tablet, lozenge or capsule) or a liquid dosage form (such as a solution or syrup), and after being placed in the mouth, they are often swallowed.

One possible disadvantage of oral administration is that pediatric and geriatric patients may have difficulty swallowing solid dosage forms. The act of swallowing the medicine often requires fluids which may not be available. Liquid dosage forms are often bad tasting and inconvenient and may increase gastric volume and the possibility of nausea and vomiting.

Moreover, there may be a substantial delay between oral administration and the therapeutic effect because the active pharmaceutical ingredient ("API") must pass through the gastrointestinal system in order to enter the blood stream; this typically takes 45 minutes or longer. In addition, API absorption through the digestive tract can be inefficient and can result in high loss due to, for example, food effects and first pass metabolism. The result is that some API(s) are impracticable for oral administration, particularly many central nervous system and many cardiovascular-acting API (s) that are intended for rapid onset in critical care situations.

In order to avoid some of the disadvantages of oral administration, injection or IV administration may be used. Injecting an API (generally intravenously), results in rapid entry of the API into the patient's blood stream. In addition, this type of delivery avoids first pass metabolism. As a result, when an API is administered parenterally, less API may be needed compared to oral administration.

However, most patients have an aversion to injections. In some patients, this aversion may be so pronounced as to make the use of injections a serious concern. Moreover, in all but unusual situations, a patient cannot self-medicate using injection or IV.

Sublingual, gingival and/or buccal administration of a drug is another oral method of administering pharmaceutical compositions. Sublingual, gingival and/or buccal medications are administered by placing them in the mouth, either under the tongue (sublingual), on gum tissue (gingival) or between the gum and the cheek (buccal). The medications dissolve and are absorbed through the mucous membranes of the mouth, where they enter into the blood stream. The sublingual, gingival and/or buccal medications are often compounded in the form of small, quick-dissolving tablets, gels, ointments, creams, sprays, lozenges, or liquid suspensions. *See* U.S. Patent Nos. 6,200,604, 6,974,590 and Shin et al., Mucoadhesive and Physicochemical Characterization of Carbopol-Poloxamer Gels Containing Triamcinolone Acetonide, 26(3) Drug Development and Industrial Pharmacy 307-312 (2000).

Lyophilized dosage forms also allow the administration of the medication through the oral mucosal tissues. *See, e.g.,* Lafon, U.S. Patent No. 4,616,047 (the '047 Patent), Nguyen et al., U.S. Patent No. 5,843,347 (the '347 Patent) and Blonde et al., U.S. Patent No. 3,855,712. These lyophilized dosage forms can be based on lyophilized emulsions such as that described in the '047 Patent or lyophilized microbeads which may then be coated with a host of compounds including polyacrylics and polymethacrylic acid esters ethyl/vinyl acetate copolymers, polymethylcyloxanes, polyacrylamides, PVP and polyvinyl acetate, polyactic and polyglycolic acids and copolymers thereof, polyurethanes, polypeptides and others as discussed in the '347 patent. In one embodiment, the coating produces a microporous semi-permeable membrane allowing the contained material to dissolve wherein an osmotic pressure is created which expels the aqueous solution containing the API. These formulations can include various diluents, sweeteners and artificial sweeteners, as well as natural or synthetic flavorings and mixtures thereof. *See also* U.S. Patent Nos. 4,490,407 and 3,939,260.

One form of lyophilized dosage form for buccal administration has been described in Caffaggi et al., Preparation and Evaluation of a Chitosan Salt-Poloxamer 407 Based Matrix for Buccal Drug Delivery, 102 Journal of Controlled Release 159-169 (2005). This article describes a compressed lyophilized oral dosage form prepared by first lyophilizing a solution containing chitosan and Poloxamer 407, deconstructing the lyophilized product into small pieces, and then using compressive force of 30kN for 1 minute in the pieces to form a compressed dosage form.

However, some dosage forms, including the lyophilized dosage forms for sublingual, gingival and/or buccal administration, can have drawbacks. They may not allow release with different rates, e.g. release of fast and sustained release of one or more API(s) or fast release of one or more API(s) and sustained release of excipients, such as flavouring/ sweetening agents administered in the same pharmaceutical composition. Additionally, they may not maintain the correct position in the mouth with the result that some of the API is swallowed, thereby reducing the oral mucosal absorption. Moreover, using compressive force to produce a dosage form adds another set of processing steps and may fundamentally change the attributes of the lyophilized product. Further, conventional lyophilized dosage forms may not allow identification, e.g. scoring or imprinting. The present invention helps address these disadvantages.

## SUMMARY OF THE INVENTION

Generally, the present invention relates to a bilayer lyophilized pharmaceutical composition aimed for oral, transoral or transmucosal absorption, and methods of making and using same.

The present invention relates to a new dosage form aimed for oral, transoral or transmucosal absorption and a process of manufacturing using the combination of different known technologies:

- lyophilization process;
- compression or compaction process;
- bioadhesion mechanism.

The dosage form according to the present invention is composed of two layers:

- an upper layer (a) that is a fast melt, rapidly dissolving, disintegrating formulation of API(s);
- a base line layer (b) formulated to adhere to the mucosa (oral mucosa), wherein layer (b) is intended for delayed, sustained or extended release of API(s) and/or excipient(s) relative to the dissolution or disintegration of layer (a).

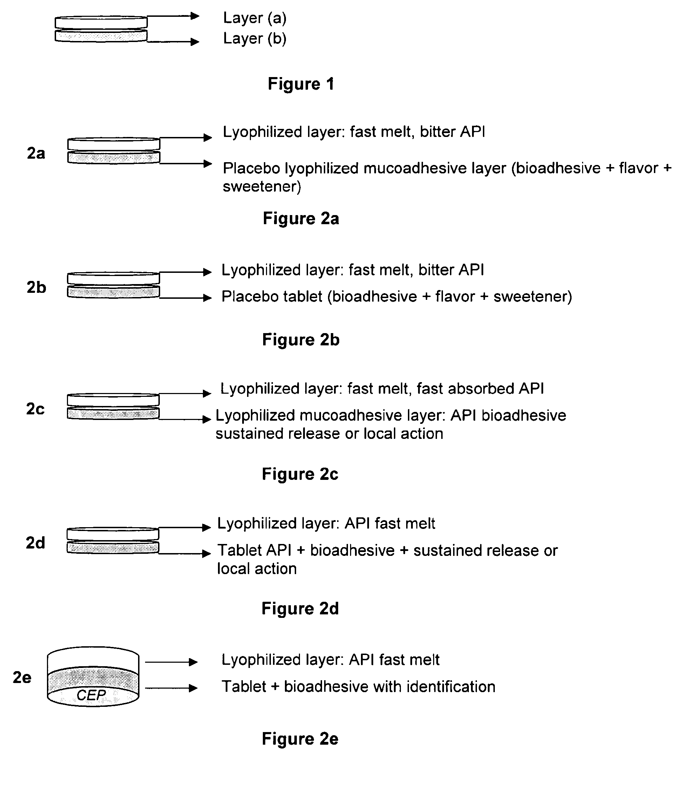

A schematic representation of the bilayer dosage form of the invention is shown on Figure 1.

By employing the present invention which allows for the oral and/or transmucosal absorption of the API(s), the API(s) may be introduced into the patient's blood stream much faster than using the oral administration route when the dosage form is swallowed, while avoiding the negative aspects of such methods.

Additionally, the bilayer dosage form of the invention allows release of API(s) and/or excipients with different rates, e.g. release of fast and sustained release of one or more API(s) and/or excipients or fast release of one or more API(s) and sustained release of excipients, such as flavouring or sweetening agents administered in the same pharmaceutical composition.

According to a preferred aspect, the bilayer dosage form of the present invention is capable of adhering to the oral mucosal tissue for a time sufficient, preferably about 10 minutes to about 180 minutes, more preferably about 15 minutes to about 180 minutes, allowing a sustained release of API(s) and/or excipients and avoidance of first pass metabolism. Alternatively, the dosage form of the present invention can also be used to deliver API(s) locally to treat conditions of the oral mucosal tissue.

In addition, by not using compressive forces in preparation of the lyophilized dosage forms of the present invention as performed by certain prior methods, the bilayer dosage forms of the present invention may allow for faster dissolution, optimized bioavailability, rapid onset of action, uniform distribution of the API(s) and/or excipients and improvement in stability.

In accordance with yet another aspect of the invention, the bilayer dosage form of the present invention may comprise a mixture of non-coated and coated API. This results in a controlled and/or extended release of API(s) since the non-coated API will quickly enter the blood stream via gastro-intestinal or transmucosal absorption, whereas the coated API will enter the blood stream at a much later time via systemic absorption.

In sum, the unique dosage form of the present invention is convenient and easy to use without water. It offers safe administration combined with rapid onset of action, sustained, controlled and/or extended release, which will result in better compliance and improvement of therapeutic efficacy.

## BRIEF DESCRIPTION OF DRAWINGS

Figures 1 and 2 represent possible arrangements of layer (a) and layer (b) of the bilayer dosage form of the invention with a bitter API as an embodiment of the invention.
Figures 2a, 2b, 2c, 2d, 2e respectively show the following arrangements:

Figure 2a : lyophilized layer (a) / placebo lyophilized mucoadhesive layer (b);
Figure 2b : lyophilized layer (a) / placebo tablet (b);
Figure 2c : lyophilized layer (a) / lyophilized mucoadhesive layer (b);
Figure 2d : lyophilized layer (a) / tablet (b) with API; and
Figure 2e : lyophilized layer (a) / tablet (b) with identification.

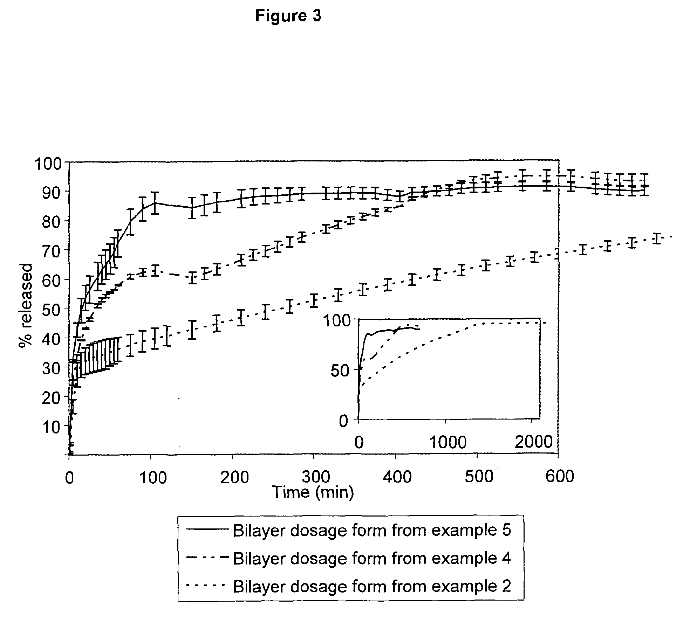

Figure 3 shows the dissolution profiles of three bilayer dosage forms according to the invention in short duration perspective and longer duration perspective (inset graph).

DETAILED DESCRIPTION

In accordance with one aspect of the invention, the bilayer dosage form of the invention comprises:

- an upper layer (a) comprising a lyophilized dosage form of API(s);
- a base line layer (b) formulated to adhere to the oral mucosa and intended for oral, transoral or transmucosal absorption, and for delayed, sustained or extended release of API(s) and/or excipient(s) relative to the dissolution or disintegration of layer (a).

A representative embodiment of the lyophilized dosage form of layer (a) is commercially available from Cephalon France as Lyoc® dosage form. The Lyoc® dosage form may be prepared in accordance with the procedures disclosed in US 4,616,047; US 5,843,347 and US 3,855,712.

The fast melt lyophilized layer (a) is directed to dissolvable or disintegrable, lyophilized dosage form adapted for direct oral dosing of API(s). It is a fast melt, rapidly dissolving or disintegrating formulation of API(s).

The lyophilized layer (a) is essentially an oral porous solid preparation that keeps all the properties - mobility or activity - of the original liquid formulation.

The composition of lyophilized layer (a) may depend on the hydrophilic or lipophilic nature of the API(s). A typical solution or suspension for preparing the lyophilized layer (a) suitable for the hydrophilic API(s) comprises:

| | |
|---|---|
| - one or more API(s) | 0.001-88% |
| - one or more filler(s) | 5-90 % |
| - one or more binder(s) | 2-20 % |
| and optionally<br>- one or more surfactant(s)<br>- one or more flavour(s) or sweetener(s); | <br>0-5 %<br>0-15 % |
| - purified water disappearing after lyophilization | QS |

According to another preferred aspect, a typical solution or suspension for preparing the lyophilized layer (a), adapted to lipophilic API(s) comprises:

| | |
|---|---|
| - one or more API(s) | 0.001-70% |
| - one or more lipid component(s) | 5-50% |
| - one or more surfactant(s) or cosurfactant(s) | 1-20% |
| - one or more binder(s) or filler(s) | 10-90% |
| And optionally - one or more flavour(s) or sweetener(s) | 0-15% |
| - purified water disappearing after lyophilization | QS |

In accordance with another aspect of the invention, the lyophilized layer (a) has a hardness from about 4,5N to about 100N.

The base line layer (b) of the bilayer dosage form of the invention may be a standard tablet or lozenge, or a lyophilized dosage form comprising bioadhesive(s) and with an extended residence time relative to the lyophilized layer (a).

Preferably, the layer (b) is a lyophilized dosage form. When (b) is in a lyophilized dosage form, it is in a form suitable to adhere to the mucosa and intended for sustained release of API(s) and/or excipients(s). This embodiment is herein referred to as "lyophilized mucoadhesive layer".

The lyophilized mucoadhesive layer (b) is a lyophilized formulation designed for transmucosal, transoral or oral absorption using lyophilization process and bioadhesion mechanism.

The lyophilized mucoadhesive layer (b) is an oral solid porous lyophilized dosage form that adheres to the oral mucosa and is intended for releasing the API(s) and/or excipient(s) for a oral and/or transmucosal absorption. Preferably, it adheres to an oral mucosal tissue for a time sufficient to release said API(s) through said oral mucosal tissue.

In accordance with one aspect of the invention, the lyophilized mucoadhesive layer (b) comprises at least one gelling agent, bioadhesive polymer and optional API(s). This dosage form preferably can adhere to an oral mucosal tissue for at least about 10 minutes to about 180 minutes, more preferably about 15 minutes up to about 180 minutes, although it needs not do so, and releases the excipient(s) and/or API(s) through the oral mucosal tissue for transmucosal absorption.

In accordance with another aspect of the invention, the lyophilized mucoadhesive layer (b) of the present invention adheres to an oral mucosal tissue to release the excipient(s) and/or API(s) through the oral mucosal tissue wherein this dosage form exhibits at least one of the following characteristics: a density of about 100 mg/ml to about 900 mg/ml, a porosity of about 10% to about 90%, and a hardness of about 4.5N (Newton) to about 100N. The lyophilized mucoadhesive layer (b) of the present invention may also exhibit two or more of the above-mentioned characteristics.

In accordance with yet another aspect, the lyophilized mucoadhesive layer (b) of the present invention results from an emulsion comprising an oil and an emulsifying agent in addition to the bioadhesive and gelling agents when a hydrophobic and/or lipophilic API(s) is used. Alternatively, when a hydrophilic API(s) is used, the lyophilized mucoadhesive layer (b) of the present invention results from a suspension comprising water, mixed with all other ingredients, including a gelling agent and a bioadhesive polymer.

Lyophilized mucoadhesive layer (b) may optionally comprise other functional excipients suitable for lyophilized dosage forms.

According to preferred embodiments, the base line layer (b) can be either:

a. a placebo composition containing a flavouring or sweetening system incorporated within bioadhesive excipients and gelling agents, lyophilized dosage form that may adhere to the oral mucosa during preferably 10-180 minutes and intended for masking the bitter taste and/or bad after-taste of the active and further more releasing a sweet and pleasant flavour;

b. a placebo tablet or lozenge with sweetening or flavouring system and bioadhesive excipients that releases a sweet and pleasant flavour after the patient swallows the dissolved or disintegrated lyophilized layer (a);

c. a lyophilized dosage form composition of active substance and bioadhesive excipients, lyophilized dosage form capable of adhering to oral mucosa during 10-180 minutes and releasing slowly the API for oral and/or transmucosal absorption and/or local action onto the mucosa;

d. a tablet containing the API, hydrophilic matrix and bioadhesive excipients for oral and/or transmucosal prolonged absorption and/or local action onto the mucosa;

e. a tablet (placebo or verum active containing) comprising bioadhesive polymer(s) with identification mark (scoring, imprinting).

Schematic representations of these combinations within the bilayer dosage form of the invention are shown on Figures 2a-2e. Options a) and c) correspond to the lyophilized mucoadhesive layer (b).

According to a preferred aspect, a typical lyophilized mucoadhesive layer (b) composition comprises:

| | |
|---|---|
| - one or more gelling agent(s) | 5-20% |
| - one or more bioadhesive polymer(s) | 5-20% |
| - one or more binder(s) or filler(s) | 20-90% |
| and optionally:<br>- one or more API(s)<br>- one or more surfactant(s)<br>- one or more flavour(s) or sweetener(s)<br>- one or more permeation enhancers | <br>0-50%<br>0-5%<br>0-15%<br>0-10% |
| - purified water disappearing after lyophilization | QS |

According to a further aspect of the invention, a typical tablet or lozenge composition for layer (b) comprises:

| | |
|---|---|
| - one or more filler(s) or binder(s) | 4-80% |
| - one or more bioadhesive polymer | 1-95% |
| - lubricant | 0.1-2% |
| and optionally:<br>- one or more API(s)<br>- one or more flavour(s) or sweetener(s) | <br>0-50%<br>0-20% |

(continued)

| - one or more buffer(s) | 0-10% |
| - one or more hydrophilic matrix | 0-95% |

The lyophilized dosage forms discussed herein exhibit the following advantages over certain non-lyophilized traditional dosage forms:

- fast and optimized disintegration or dissolution;
- optimized bioavailability or bioequivalence with rapid onset of action;
- convenience and reliability of administration without the need for additional water, improvement of compliance and efficacy,
- potential improvement of tolerance as a result of the fine dispersion of particles into saliva, this avoids direct adhesion of drug to the gastrointestinal tract mucosa;
- potential improvement of stability of sensitive products;
- sugar-free, salt-free: suitable for all diet.

According to a further aspect, the present invention also provides a process for preparing the bilayer dosage form of the invention.

According to a first embodiment, the process of preparation a bilayer dosage form according to the present invention comprises the steps of:

i. providing a suspension suitable for preparing the lyophilized layer (a),
ii. providing layer (b) in the form of a tablet, lozenge or a suspension suitable for preparing the lyophilized mucoadhesive layer (b),
iii. adding said suspension suitable for preparing the lyophilized layer (a) on said layer (b) in the form of a tablet, lozenge or a suspension suitable for preparing the lyophilized mucoadhesive layer (b),
iv. freezing the bilayer system obtained,
and
v. lyophilizing.

According to a preferred aspect, said suspension suitable for preparing the lyophilized mucoadhesive layer (b) is obtained by:

(a) dissolving a gelling agent in water at a temperature below 30°C, preferably 10°-25°C,
(b) dissolving a bioadhesive polymer, optional API(s) and the other excipients in water at a temperature below 100°C, preferably 30°-80°C, more preferably 50°-70°C,
(c) mixing said suspensions from steps (a) and (b), and
(d) optionally adding API(s) to said suspension from step (c) and mixing until the suspension is substantially homogeneous.

Generally, step (c) of mixing is performed at a temperature between about 10°C and about 30°C, preferably at a temperature between about 15°C and about 25°C.

According to another preferred aspect, said suspension suitable for preparing the lyophilized mucoadhesive layer (b) is obtained by:

(a) preparing an aqueous suspension containing at least one hydrophilic compound,
(b) preparing an oil suspension containing at least one lipophilic compound, and
(c) mixing said aqueous suspension phase of step (a) and said oil suspension from step (b) to form an emulsion.

Said hydrophilic compound is generally chosen from a gelling agent, bioadhesive polymer, binder, filler and optional hydrophilic API(s); said lipophilic compound is generally chosen from an emulsifying agent, sweetener, flavoring agent and optional lipophilic API(s).

Prior to adding the suspension suitable for preparing the lyophilized layer (a), the suspension suitable for preparing the lyophilized mucoadhesive layer (b) or tablet or lozenge of layer (b) may be cooled at a temperature preferably between -20°C and +10°C.

When layer (b) is a lyophilized mucoadhesive layer said layer (b) can be added to layer (a) previously cooled at a temperature preferably between -20°C and +10°C.

The suspension suitable for preparing the lyophilized layer (a) is obtained by preparing a liquid preparation of the API(s) and optional further ingredients as defined above. The API(s) is first dissolved or dispersed in a liquid phase consisted of water or water with surfactant(s) or a system composed of oil, water and surfactant(s). The dispersion of API(s) in the liquid phase may be optimized, if required. Optimization of dispersion of API(s) is obtained by using adequate surfactant(s) and homogenizing. A verification of homogeneity can be made by appropriate methods of sampling and dosing known by the skilled person.

Generally, the tablet, lozenge, lyophilized mucoadhesive layer (b), suspension suitable for preparing the lyophilized mucoadhesive layer (b), lyophilized layer (a) or the suspension suitable for preparing the lyophilized layer (a), as the case may be, is deposited into a mould prior to adding the other component.

Preformed moulds that can be used include containers such as blisters. Preferred for the invention are blisters. Suitable mould and blister materials include PVC, PVC/PVDC, Aclar or Aluminium foil.

Layer (b) in the form of a tablet or a lozenge may be prepared by any methods known to one skilled in the art. Generally, it is prepared by direct compression or compaction of a mixing of powder composed of optional API(s) and excipients by using flat punches. The punches can be scored with identification mark. The tablet or lozenge will preferably have a thickness of 1-2 mm.

The process of the invention according to anyone of the embodiments discussed above may further comprise the step of sealing the mould containing the obtained bilayer dosage form.

Preferably, the freezing steps mentioned above are carried out at temperature comprised between -45°C and -15°C. The active molecules are immobilized; their properties remain unaltered as the rate of chemical reactions is nearly zero at this low temperature.

The freeze-drying steps (or sublimation) mentioned above are carried out at low temperature, preferably comprised between -25°C and 25°C and under low pressure, preferably comprised between 200 and 500 microbar, where ice is directly converted into vapour phase. A secondary drying is preferably operated at a temperature comprised between 25°C and 50°C during 60 to 120 minutes at a pressure comprised between 10 and 100 microbar.

In accordance with another aspect, the lyophilized mucoadhesive layer (b) results from an emulsion comprising an oil and an emulsifying agent in addition to the bioadhesive and gelling agent when a hydrophobic and/or lipophilic API(s) is used. Alternatively, when a hydrophilic API(s) is used, the lyophilized mucoadhesive layer (b) of the present invention results from a suspension comprising water, mixed with all other ingredients, including a gelling agent and a bioadhesive polymer.

In accordance with yet another aspect of the invention, there is also provided the use of a bilayer dosage form of the invention for the preparation of a medicament suitable for (a) being placed into intimate contact with an oral mucosal tissue of a patient in need of said medicament; and (b) adhering to the oral mucosa and releasing the therapeutically effective amount of the at least one API(s) through the oral mucosal tissue.

Once the bilayer dosage forms of the present invention are placed on a specific location of the oral mucosal tissue, the bioadhesive polymer helps the dosage form to remain at the correct position for an extended period of time to aid in the sustained release of excipients and/or API(s) through the oral mucosal tissue, thereby increasing the transmucosal absorption of the excipients and/or API(s), avoiding first pass metabolism.

Additionally, at the same time, the gelling agent and/or hydrophilic matrix in the lyophilized mucoadhesive layer (b) reacts with the saliva in the mouth and immediately swells by formation of a hydrated network. This swelling of the dosage form allows to provide a large adhesive surface for maximum contact with the oral mucosal tissue. Moreover, the network created by hydration of the dosage form in contact with the oral mucosa allows maintaining a physical structure compatible with a residence time of the dosage form in the mouth.

The frequency of dosing depends on various factors including the amount of API(s) present in the dosage form, the size of the dosage form, the weight of the patient, the condition of the patient, side effects of the API, etc. The administration of multiple dosage forms and multiple frequency of dosing is contemplated depending upon the above factors as well as duration of the patient's condition, how long the API(s) stays in the patient's system, etc.

While the specification concludes with the claims particularly pointing and distinctly claiming the invention, it is believed that the present invention will be better understood from the following description.

All percentages and ratios used herein are by weight of the total composition and all measurements made are at 25°C and normal pressure unless otherwise designated. All temperatures are in degrees Celsius unless specified otherwise.

The present invention can comprise (open ended) or consist essentially of the components of the present invention as well as other ingredients or elements described herein.

As used herein, "comprising" means the elements recited, or their equivalent in structure or function, plus any other element or elements which are not recited.

The terms "having" and "including" are also to be construed as open ended unless the context suggests otherwise.

As used herein, "consisting essentially of" means that the invention may include ingredients in addition to those recited in the claim, but only if the additional ingredients do not materially alter the basic and novel characteristics of the claimed invention. Preferably, such additives will not be present at all or only in trace amounts. However, it may be possible to include up to about 10% by weight of materials that could materially alter the basic and novel characteristics of the invention as long as the utility of the compounds (as opposed to the degree of utility) is maintained.

All ranges recited herein include the endpoints, including those that recite a range "between" two values.

Terms such as "about," "generally," "substantially," and the like are to be construed as modifying a term or value such that it is not an absolute, but does not read on the prior art. Such terms will be defined by the circumstances and the terms that they modify as those terms are understood by those of skill in the art. This includes, at very least, the degree of expected experimental error, technique error and instrument error for a given technique used to measure a value.

Note that while the specification and claims may refer to a tablet or other dosage form of the invention as, for example, containing particles having a certain particle size or distribution, it may be difficult to tell from the final dosage form that the recitation is satisfied. However, such a recitation may be satisfied if the materials used prior to final blending and tablet formulation, for example, meet that recitation. Indeed, as to any property of a dosage form which cannot be ascertained from the dosage form directly, it is sufficient if that property resides in the formulation just prior to producing a dosage form therefrom.

In describing the preferred embodiments of the present invention, specific terminology will be resorted to for the sake of clarity. However, the invention is not intended to be limited to the specific terms so selected, and is understood that each specific term includes all technical equivalence which operate in a similar manner to accomplish the same purpose.

The terms "lyophilized solid dosage form(s)" are defined as solids such as tablets, caplets, wafers, films and the like which result from lyophilization and are adapted to adhere to the oral mucosal tissue. In particular, these terms may refer to the layer (b) of the bilayer dosage form of the invention.

The terms "oral mucosa" and "oral mucosal tissue" in accordance with the present invention includes the mucous membrane that covers all structures inside the oral cavity except the teeth, including the tissue under the tongue, gum tissue, between the gum and the cheek, and cheek pouch.

The terms "sustained release" in this disclosure means that the dosage form begins its release and continues that release over an extended period of time, that is, from 10 minutes to about 180 minutes, more preferably about 15 minutes to about 180 minutes. Release can occur beginning almost immediately or can be delayed. Release can be constant, can increase or decrease over time, can be pulsed, can be continuous or intermittent, and the like.

"Controlled release" in accordance with the present invention means a release of the active pharmaceutical ingredient ("API") at a particular desired point in time once the dosage form has been placed into the mouth of a patient. Generally, it involves a delayed but otherwise complete release. A sudden and total release in the stomach at a desired and appointed time or a release in the intestines such as through the use of an enteric coating, are both considered controlled release, as opposed to a passive release dosage form wherein within moments of reaching the stomach, the API(s) begins to be exposed to digestion and possibly the absorption process (such as occurs when swallowing a raw powdered API). Controlled release requires that release occur at a predetermined time or in a predetermined place within the digestive tract. It is not meant to be a passive, uncontrolled process as in swallowing a normal tablet.

An "extended release" dosage form is a dosage form which generally begins its release and continues that release over an extended period of time. Release can occur beginning almost immediately or can be delayed, in which case the extended release dosage form is also a controlled release dosage form as described above. Release can be constant, can increase or decrease over time, can be pulsed, can be continuous or intermittent, and the like. Generally, however, the release of the API(s) from an extended release dosage form will exceed the amount of time of release of the drug taken as a normal, passive release tablet. Thus, for example, while all of the API(s) of an uncoated aspirin tablet should be released within, for example, four hours, an extended release dosage form could release a smaller amount of aspirin over a period of six hours, 12 hours, or even longer. Extended release in accordance with the present invention generally means that the release occurs for a period of six hours or more, and more preferably 12 hours or more.

As used herein, the term "mould" refers to any containment of any shape and size suitable for the dosage form prepared according to the invention and capable of accommodating said dosage form during its preparation.

As used herein, the term "suspension" also encompasses a solution, dispersion, emulsion, gel and the like. In accordance with the present invention, as long as the ingredients, such as a gelling agent, a bioadhesive polymer and optionally an API, are mixed in a solvent in a substantially homogeneous manner, it is not critical that these ingredients are fully or partly suspended, dissolved, dispersed, emulsified, gelled and the like in the solvent before the lyophilization step.

The term "oil" in this disclosure includes anything that can form an oil phase in an emulsion.

Active pharmaceutical ingredient(s) ("API(s)") which may be used in accordance with the present invention include materials that are biologically or therapeutically active, capable of being lyophilized. The API in the layer (a) is intended for ingestion or absorption by swallowing. When an API(s) is also present in layer (b), it may be orally and/or transmucosally absorbed. The API(s) in accordance with the present invention may include systematically distributable API(s), vitamins, minerals, dietary supplements, or mixtures thereof, as well as nonsystematically distributable API(s). In this disclosure, API(s) may be comprised of either a mixture of non-coated and coated API(s), or non-coated API(s) alone.

Generally, coating of an API(s) can be accomplished using, for example, a Wurster fluidized bed where the coating material enters from the bottom of the reactor. See also the techniques described in U.S. Patent Nos. 6,024,981, 4,949,588, 5,178,878, and 5,055,306. See also Lieberman, Pharmaceutical Dosage Form: Tablets Vol. 1 (2d ed. 1989), 732-36. Other coating techniques contemplated by the present invention include spray drying, spraying chilling, spray congealing, hot melt coating in fluid beds or through extrusion, fluid bed top spray mode, fluid bed tangential spray mode, conventional pan coating, perforated pan coating, micro-encapsulation through coacervation or other forms of phase separations, interfacial polymerization, supercritical fluid coating, spinning disc coating and other centrifugation coating techniques.

In this disclosure, the term "coating" is used broadly to include any material mixed, granulated, agglomerated, deposited on or coated with the API.

Generally, the coating is made from any natural or synthetic materials including: acrylic polymers, modified celluloses, cellulosic polymers, lactic acid polymers, glycolic acid polymers and copolymer thereof, and the like, fatty acids, fatty acid esters, fatty alcohols, phospholipids, hard fat, waxes, hydrogenated vegetable oils, and the like.

These natural or synthetic materials can be pH dependant materials. In one embodiment, the coating materials will become soluble at a pH which is generally acidic (pH 7 or below) and in another embodiment, the coating materials will become soluble at a pH which is generally basic (pH 7 or above). In a third embodiment, the materials become soluble at a generally neutral pH (pH 6-8). There are a number of factors which determine which material is used as a coating in a given situation including, without limitation, ease of handling and processing, cost, thickness, site of intended dissolution of the coating and/or the API, and the type and pH of the lyophilization solvent to be used.

In one preferred embodiment, the coating is made from a material that becomes soluble at a pH of about 6.5 or below. In another embodiment, the coating becomes soluble at a pH of between about 6.0 and about 6.5. These polymers and copolymers should preferably be pharmacologically acceptable, capable of providing appropriate release and while still being convenient to process. These include, for example, acrylic polymers, modified cellulose amino alkyl acrylate copolymers such as, for example, copolymers of methylmethacrylate, butylmethacrylate and dimethylaminoethyl methacrylate.

A particularly preferred material can be obtained under the mark Eudragit® E-100, which can be used in normal form or in micronized Eudragit® E-100 and mixtures thereof. Eudragit® is a trademark of Rohm GmbH, Chemische Fabrik, Kirschenallee, D-64293, Darmstadt, Germany for a group of acrylic polymers.

These materials are generally solid at room temperature. However, they may be applied by being dissolved, suspended, emulsified, dispersed or the like in a solvent or solvent system, such as water or a supercritical fluid.

The coated API(s) may be in the form of, without limitation, grains, microparticles, crystals, granules (wet or dry granulation), microgranules, agglomerates, pellets, mini-tablets, beads, solid supports, microcrystals and powders

API(s) include, without limitation, antalgic, anesthetic, antacids, analgesics, anti-inflammatories, antibiotics, antidiabetes, diuretics, anorexics, antihistamines, antiasthmatics, antidiuretics, antiflatulents, antimigraine agents, antispasmodics, sedatives, antihyperactives, anti-hypertensives, tranquilizers, decongestants, immunosuppressants, anticancers, antivirals, antiparasitics, antifungals, antiemetics, anti-depressants, antiepileptics, local anesthetics, vasoactive agents, antiasthmatics, skeletal muscle relaxants, drugs for parkinsonism, antipsychotics, hematopoietic growth factors, antihyperlipidemics, anticoagulants, fibrinolytics, chemotherapeutic agents, antithrombotics, hormones, therapeutic proteins and peptides, antiarrhythmia, antiangina, beta blockers, calcium inhibitors, drugs intended for management of moderate to sever pain when a continuous analgesic is needed for an extended period of time, drugs for treatment of anxiety, depression, emesis, migraine, panic attack, attention deficit hyperactivity disorder (ADHD), narcolepsy, fatigue, apnea, seizure, and cardiovascular disorders and mixtures thereof. Of these, the preferred API(s) are antalgics, anaesthetics, anti-inflammatories, chemotherapeutic agents, proteins and peptides, drugs intended for management of moderate to severe pain when a continuous analgesic is needed for an extended period of time, drugs for treatment of anxiety, depression, emesis, migraine, panic attack, attention deficit hyperactivity disorder (ADHD), seizure, cardiovascular disorders, betablocker, and calcium inhibitors. Of these, the most preferred API(s) are fentanyl, modafinil, ondansetron, granisetron, sumatriptan, tramadol, desmopressin, calcitonin, insulin, GLP-1, PPY, oxycodone, taxol, clarithromycin, erythromycin, azithromycin, amoxicillin, fexofenadine, ibuprofen, phenitol, acyclovir, famciclovir, DOPA, and L-DOPA and as well as their salts.

As used in this disclosure, the term "vitamin" refers to organic substances that are required in the diet. For the purposes of the present invention, vitamin(s) include, without limitation, thiamin, riboflavin, nicotinic acid, pantothenic acid, pyridoxine, biotin, folic acid, vitamin B12, lipoic acid, ascorbic acid, vitamin A, vitamin D, vitamin E and vitamin K. Also included within the term vitamin are the coenzymes thereof. Coenzymes are specific chemical forms of vitamins.

Coenzymes that may be useful in the present invention include thiamine pyrophosphates (TPP), flavin mononucleotide (FMM), flavin adenine dinucleotide (FAD), nicotinamide adenine dinucleotide (AND), nicotinamide adenine dinucleotide phosphate (NADP) coenzyme A (CoA) pyridoxal phosphate, biocytin, tetrahydrofolic acid, coenzyme $B_{12}$, lipoyllysine, 11-cis-retinal, and 1,25-dihydroxycholecalciferol. The term vitamin(s) also includes choline, carnitine, and alpha, beta, and gamma carotenes.

As used in this disclosure, the term "mineral" refers to inorganic substances, metals, and the like required in the human diet. Thus, the term "mineral" as used herein includes, without limitation, calcium, iron, zinc, selenium, copper, iodine, magnesium, phosphorus, chromium and the like and mixtures thereof.

While having one API is desired, multiple API(s) may also be used. The amount of API(s) used can vary greatly and can depend upon, among other things, the type and properties of the API, the condition it is intended to treat, the method of administration, the size and type of the patient, the size and nature of the dosage form, whether or not more than one API is to be delivered from the dosage form and how many dosage forms will be used to deliver each dose.

Generally, the total amount of API(s) for any individual dosage form is a therapeutically effective amount. The terms "therapeutically effective amount" are the amount or quantity of a drug or API(s) which is sufficient to elicit the required or desired therapeutic response, or in other words, the amount which is sufficient to elicit an appreciable biological response when administered to a patient. This does not need to be optimal, nor even provide a cure or symptomatic relief.

As used with reference to a vitamin or mineral, the terms "effective amount" mean an amount at least about 10% of the United States Recommended Daily Allowance ("RDA") of that particular ingredient for a patient. For example, if an intended ingredient is vitamin C, then an effective amount of vitamin C would include an amount of vitamin C sufficient to provide 10% or more of the RDA.

In general, the total amount of API(s) in a single dosage form is in an amount of about 0.001 mg to about 1.5 g by weight, more preferably about 0.01 mg to about 0.5 g, even more preferably about 0.02 mg to about 200 mg, and most preferably about 0.05 mg to about 100 mg. This is based on the amount of API(s) only - not counting, for example, the amount of other excipients, coatings and the like in the final dosage form.

In terms of the proportion of the API(s) with respect to the total weight of the dosage form, the amount of API (s) can range from about 0.001 % to about 88% by weight, and more preferably from about 0.1% to about 30% by weight relative to the total weight of the dosage form.

The API(s) may be hydrophilic, hydrosoluble, lipophilic, or liposoluble. In this disclosure, the term "hydrosoluble" is used interchangeably with the term "hydrophilic." Similarly, the term "liposoluble" is used interchangeably with the term "lipophilic." Moreover, the term "hydrophobic" is used interchangeably with the terms "liposoluble," lipophilic" and "water insoluble" in this disclosure.

The average particle size of hydrophilic API(s) ranges from 10 $\mu$m to 800 $\mu$m, more preferably from 100 $\mu$m to 500 $\mu$m. The particle size is measured by sieving based on a determination by weight using the following screens: 1000, 710, 500, 355, 250, 180, 125, 90 $\mu$m and the pan. In these instances, no more than about 35% by weight would go through a 90 $\mu$m screen. The particle size of the API(s) is measured before being exposed to a liquid used in the manufacturing process. The particle size of less than 20 $\mu$m is measured by laser light scattering, using for example a Coulter LS230.

The average particle size of lipophilic API(s) ranges from 1 $\mu$m to 100 $\mu$m, more preferably from 2 $\mu$m to 20 $\mu$m. The particle size is measured by laser light scattering, using for example a Coulter LS230.

The hydrophilic matrix according to the present invention includes any hydrophilic system suitable for controlled drug delivery in solid oral dosage, such as cellulose and derivatives. Preferably, it includes hydroxypropyl-methylcellulose (HPMC) polymers, hypromellose, methyl cellulose and Methocel® (available from Colorcon Ltd., Dartford, Kent, England).

Gelling agents in accordance with the present invention include materials capable of forming a gel and make the solution, suspension or emulsion viscous before the lyophilization step. Such gelling agents are used to avoid aggregation, agglomeration or cohesion of small insoluble API particles before lyophilization. Gelling agents are also often solvent swellable when in contact with water or saliva in the mouth. They provide a large adhesive surface for maximum contact with the oral mucosal tissue. Also, presence of these gelling agents confers a gel-like structure upon hydration of the dosage form in the mouth. This gel-like structure is useful for maintaining the dosage form during the time needed for permeation of drugs through the mucosa. Moreover, this gel-like structure gets the dosage form softer when in place on the oral mucosa, what is comfortable for the patient.

Such gelling agents may include, without limitation, acacia, alginic acid, bentonite, carbomer, carboxymethylcellulose sodium, cetostearyl alcohol, colloidal silicon dioxide, ethylcellulose, gelatin, hydroxy-ethylcellulose, hydroxypropyl cellulose, hydroxypropyl methylcellulose, magnesium aluminum silicate, maltodextrin, methylcellulose, polyvinyl alcohol, povidone, propylene carbonate, propylene glycol alginate, sodium alginate, sodium starch glycolate, starch tragacanth, polysaccharides, such as colloid, hydrocolloid, guar gum, arabic gum, tragacanth gum, xanthan gum, carraghenans, pectins, starch, and specialized polymers such as poloxamer 188, poloxamer 407, poloxamine, acrylic polymers, carbopol and mixtures thereof.

Of these, the preferred gelling agents are colloids or hydrocolloids of polysaccharides, such as, guar gum, arabic gum, tragacanth gum, xanthan gum, carraghenans, pectins, starch, and specialized polymers such as poloxamer 188, poloxamer 407, poloxamine, acrylic polymers, and carbopol.

The gelling agents can be used in conventional amounts and preferably in an amount of from about 5% to about 20% by weight relative to the total weight of the dosage form.

A bioadhesive polymer in accordance with the present invention includes materials capable of adhering to a biological substrate and remaining there for an extended period of time to aid in sustained release of an API(s) through oral mucosa membrane. The term, bioadhesive, in accordance with the present invention, includes compounds conventionally referred to as muco-adhesive, and these terms may be used interchangeably herein.

Such bioadhesive polymers may include, without limitation, polycarbophil, polyacrylates, cellulose, cellulose derivatives, carboxymethyl cellulose, sodium carboxymethyl cellulose, methyl cellulose, hydroxypropyl cellulose, hydroxy propyl methyl cellulose, hydroxy ethyl cellulose, hyaluronic acid, sodium alginate, povidone, chitin, glycerol ester, pectin, gelatin, PEG and mixtures thereof.

Of these, the preferred bioadhesive polymer is cellulose, cellulose derivatives or mixtures thereof.

The bioadhesive polymer can be used in conventional amounts and preferably in an amount of from about 1% to about 95% by weight, and more preferably from about 5.0% to about 20% by weight relative to the total weight of the dosage form.

A solvent in accordance with the present invention includes water such as filtered, purified, distilled water or a mixture thereof.

In another preferred embodiment, the lyophilized mucoadhesive layer (b) of the present invention may further comprise an oil and an emulsifying agent, when the API(s) is a lipophilic API. The emulsion can be an oil-in-water or a water-in-oil-in-water emulsion (double emulsion).

Oils are used in accordance with the present invention as a carrier of a hydrophobic and/or lipophilic API. Some oils that may be useful in the present invention include, without limitation, Miglyol® 810-818 (available from Sasol, Marl, Germany), mono, di-triglycerides esters, fatty acid of glycerol, mineral oil, vegetable oil and a reformed vegetable oil of a known composition. Of these, the preferred oils are Miglyol® 810-818, mono-di-triglycerides esters, fatty acid esters of glycerol. Oils can be used in conventional amounts and preferably in an amount of from about 0.5% to about 40% by weight, and more preferably from about 2% to about 20% by weight relative to the total weight of the dosage form.

Emulsifying agents are used to prepare an oil-in-water or water-in-oil-in-water emulsion for lyophilization. Emulsifying agents are used in accordance with the present invention as wetting agents that lower the interfacial tension between oil and water, allowing easier spreading. Non-limiting examples of emulsifying agents that are suitable for the present invention are anionic, cationic, amphoteric, nonionic surfactants, such as dioctyl sulfosuccinate, sucroester 7, sucroester 11, sucroester 15, polysorbate 20, polysorbate 60, polysorbate 80; poloxamers 188, 407, sorbitan stearate, polyethoxyethers of fatty glycerides, cetyl ethers of polyoxy-ethylenglycol, palmitostearate of polyoxyethylene-glycol, lecithins, and mixtures thereof. Some emulsifying agents that may be useful in the present invention include, without limitation, various grades of the following commercial products: Arlacel® (available from Uniquema, New Castle, DE, USA), Tween® (available from Cocgni, Deutschland GMBH and Co, Monheim am Rhein, Germany), Capmul® (available from Abitec Corporation, Janesville, WI, USA), Centrophase® (Ultimate Source, Dallas, TX, USA), Cremophor® (available from BASF, Ludwighafen, Germany), Labrafac™, Labrafil®, Labrasol™ (available from Gattefossé, Saint-Priest, France), Myverol™ (available from Uniquema, New Castle, DE, USA), Tagat® (available from Goldschmidt AG, Essen, Germany), Simulsol® (available from Seppic, Paris, France), Solutol® (available from BASF, Ludwighafen, Germany), Softigen® (available from Sasol, Marl, Germany), and any non-toxic short and medium chain alcohols. Of these, the preferred emulsifying agents are polysorbate 20, polysorbate 60, polysorbate 80, sucroester 7, sucroester 11, sucroester 15, sorbitan, poloxamer and dioctyl sulfosuccinate, laurylsulfate. Emulsifying agents can be used in conventional amounts and preferably in an amount of from about 0.01 % to about 20% by weight, and more preferably from about 0.1 % to about 10% by weight relative to the total weight of the dosage form.

The layers of the present invention may further comprise at least one excipient, which is generally a pharmaceutically inactive substances added to the formulation to facilitate tableting or lyophilization. Generally, layers of the present invention may contain excipients for various reasons, such as to bulk up the amount of solids, to buffer the product, to taste-mask the bad or bitter tasting API(s), to facilitate the process by avoiding foam forming, to increase the rate at which the API(s) permeate through the oral mucosal tissue and enter the blood stream and/or to protect the API (s) from the adverse effects of freezing and/or drying, etc. Although certain excipients may be transmucosally absorbed to some extent because of their chemical nature, many of the excipients would be released in the oral cavity to be swallowed. Examples of some excipients used in accordance with the invention, without limitation, are binders, fillers, sweeteners, flavoring agents, permeation enhancers, buffers, coloring agents, defoaming agents, mixtures thereof, and the like. Of these, binders and fillers are hydrophilic excipients. Sweeteners, flavoring agents, coloring agents, defoaming agents and permeation enhancers may be either hydrophilic or lipophilic, depending on the specific compound.

Binders and fillers are used to contribute to the bulk and stability of the matrix and control the rate at which the matrix will dissolve. Binders can be anything known to be used as a binder. Binders that may be useful in the present invention include Dextran 70, povidone 12, povidone 17 or povidone 30, copovidone, gelatin, starch, pregelatinized starch, methyl cellulose, hydroxypropylmethyl cellulose and mixtures thereof. Binders can be used in conventional amounts and preferably in an amount of from about 5% to about 90% by weight, and more preferably from about 10% to about 90% by weight relative to the total weight of the dosage form.

Fillers can be anything known to be useful as fillers. Some fillers that may be useful in the present invention include mannitol, lactose, sorbitol, isomalt, glycine, cyclodextrins and derivatives, glucose, sucrose maltodextrine, trehalose. Fillers can be used in conventional amounts and preferably in an amount of from about 0.5% to about 90% by weight, and more preferably from about 5% to about 50% by weight relative to the total weight of the dosage form.

Permeation enhancers as used herein are materials that can increase the rate at which the API(s) permeates through the oral mucosal tissue and enters the blood stream. Such permeation enhancers may include, without limitation, sodium salicylate, sodium dodecyl sulfate, citric acid, sodium bicarbonate, sodium carbonate, sodium glycolate, sodium deoxycholate, sodium taurocholate, fatty acids, Azone®, chitosan and its derivatives, terpenoids, protease inhibitors and a mixture thereof. Permeation enhancers can be used in conventional amounts and preferably in an amount of from about 0.001% to about 10% by weight, and more preferably from about 0.01% to about 5% by weight relative to the total weight of the dosage form.

Buffers as used herein are materials capable of adjusting pH in the preparation for the purposes of stability, permeation and/or taste. Some buffers that may be useful in the present invention include any weak acid or weak base or, preferably, any buffer system that is not harmful to the oral mucosa and gastrointestinal system. These include, but are not limited to, any of the acids or bases such as monobasic potassium phosphate, anhydrous dibasic sodium phosphate, sulfuric acid, sodium hydroxide, sodium carbonate, potassium carbonate, disodium hydrogen phosphate, sodium dihydrogen phosphate, and the equivalent potassium salts. Of these, the preferred buffers are monobasic potassium phosphate, anhydrous dibasic sodium phosphate, sulfuric acid, sodium hydroxide, citric acid, sodium citrate, acetic acid, sodium acetate, sodium carbonate, sodium bicarbonate or a mixture thereof. Buffers can be used in conventional amounts and preferably in an amount of from about 0.1 % to about 10% by weight, and more preferably from about 0.2% to about 5% by weight relative to the total weight of the dosage form.

Sweeteners include both natural and artificial sweeteners that are known to be and can be used as sweeteners. Some natural sweeteners that may be useful in the present invention, without limitation, include sucanat, (unpasteurized) honey, frozen juice concentrates, dates, raisin, fructose, and mixtures thereof. Some artificial sweeteners that may be useful in the present invention, without limitation, include saccharin such as sodium saccharin, cyclamate, aspartame, sucralose, neotame, and acesulfam potassium and polyols. Sweeteners may be used in conventional amounts, and preferably in an amount ranging from about 0.1 % to about 20% by weight, and more preferably from about 0.5% to about 10% by weight relative to the total weight of the dosage form.

Flavoring agents can be anything known to be useful as flavoring agents. Flavoring agents that may be useful in the present invention may include synthetic flavor oils and flavoring aromatics and/or natural oils, extracts from plants, leaves, flowers, fruits and mixtures thereof. These may include cinnamon oil, oil of wintergreen, peppermint oils, clove oil, bay oil, anise oil, eucalyptus, thyme oil, cedar leave oil, oil of nutmeg, oil of sage, oil of bitter almonds and cassia oil and mixtures thereof. Also useful as flavoring agents are vanilla, citrus oil, including lemon, orange, banana, grape, lime and grapefruit, and fruit essences, including apple, pear, peach, strawberry, raspberry, cherry, plum, pineapple, apricot and mixtures thereof. Flavoring agents may be used in conventional amounts, and preferably in an amount ranging from about 0.1 % to about 20% by weight, and more preferably from about 1% to about 10% by weight relative to the total weight of the dosage form.

Coloring agents can be anything known to be used as a coloring agent. Coloring agents useful in the present invention may include titanium dioxide, and dyes suitable for food such as those known as F. D. & C. dyes and natural coloring agents such as grape skin extract, beet red powder, beta-carotene, annatto, carmine, turmeric, paprika, and mixtures thereof. Coloring agents may be used in conventional amounts, and preferably in an amount ranging from about 0.01% to about 5% by weight, more preferably from about 0.05% to about 2% by weight relative to the total weight of the dosage form.

Defoaming agents can be any substance used to reduce or inhibit foam formation caused by, for example, bioadhesive polymers, emulsifying agents and/or flavoring agents during the mixing process. Defoaming agents useful in the present invention may include silicone emulsions, simethicone, dimethicone copolyol, glyceryl oleate, propylene glycol, poloxamer (PO-EO block copolymers). Defoaming agents may be used in conventional amounts, and preferably in an amount ranging from about 0.001 % to 5% by weight, more preferably from 0.005% to 3 % by weight relative to the total weight of the dosage form.

The lyophilized layers of the present invention are prepared without any step of breaking, cutting, milling or pulverizing the lyophilized product and/or compressing the broken, cut, milled or pulverized lyophilized materials into tablets by applying compressive force. This results in a lyophilized solid dosage form having, for example, a hardness of from about 4.5N to about 100N, allowing a much softer and flexible dosage form than compressed tablets of the prior art. The softness of the lyophilized solid dosage forms of the present invention helps reduce the adverse feeling in the oral mucosa cavity on use and therefore results in better patient compliance.

The hardness of the lyophilized layers of the present invention is tested using a tablet hardness meter manufactured by Schleuniger Pharmatron. This is an electrically operated tablet tester that eliminates operator variability. The tests are performed with 10 tablets and the mean value is determined. Tablet hardness is represented by the force needed to crush the tablet (units kp or N). A larger number indicates a stronger tablet.

Moreover, the lyophilized mucoadhesive layer (b) of the present invention may have a porosity between about 10% to about 90%. This is also an advantage over some prior art dosage forms since it allows for fast dissolution, rapid gelling and adhesion, and rapid onset of action.

The porosity of the lyophilized mucoadhesive layer (b) of the present invention is calculated by the following equation:

$$\text{Porosity (\%)} = \frac{\text{Volume of water used prior to lyophilization}}{\text{Volume of the final dosage form after lyophilization}} \times 100$$

Even though the entire amount of water in the dosage form completely sublimes thereby leaving pores in the final dosage form, the volume of the dosage form after lyophilization does not change. Therefore, the porosity is determined by the percentage of water in the dosage form before lyophilization.

The lyophilized mucoadhesive layer (b) of the present invention may have a density ranging from about 100 mg/ml to about 900 mg/ml. During the lyophilization process, the volume of dosage form remains unchanged. Therefore, the volume of the final dosage form is similar to the volume of the suspension before lyophilization.

Accordingly, the density of the final lyophilized mucoadhesive layer (b) after lyophilization is calculated using the following equation:

$$\text{Density} = \frac{\text{(weight of suspension} - \text{weight of water)}}{\text{Volume.}}$$

In yet another preferred embodiment, the layer (b) of the present invention may be a controlled released and/or extended release formulation containing a mixture of coated and non-coated API(s). The non-coated API(s), which are explained in details above, will be absorbed through the mucous membranes of the mouth, where they enter into the blood stream, and therefore will be fast-acting. On the other hand, the coated API(s) will be swallowed and passed through the gastrointestinal system in order to enter the blood stream, and therefore will take much longer to deliver therapeutic effect. Accordingly, a combination of coated and non-coated granules in the bilayer dosage form of the present invention will result in an extended release of the API.

As long as a resultant dosage form meets the overall objectives of the present invention, and contains a therapeutically effective amount of at least one API, the shape or size of the dosage form is not critical. In fact, the dosage form can be of any shape, such as a circle, oval, rectangle, square, triangle, octagon and etc.

However, it is preferred that the layer (b) of the present invention has a maximum diameter of about 3 to about 40 mm and a thickness of about 1 to about 7 mm, preferably a maximum diameter of about 5 to about 20 mm and a thickness of about 1 to about 3 mm, as measured by a caliper rule.

Preferably, the bilayer dosage form of the present invention has a maximum diameter of about 5 to 20 mm, preferably 8 to 16 mm, and a thickness of about 2 to 8 mm.

In a preferred embodiment, the lyophilized mucoadhesive layer (b) of the present invention is prepared by first preparing a suspension containing a gelling agent and a bioadhesive polymer. This is preferably carried out by suspending, dissolving, or dispersing the bioadhesive polymer, preferably in water, at a temperature below 100°C, preferably at about 30°C-80°C, preferably 50°C-70°C, and then in a separate container, suspending, dissolving, or dispersing the gelling agent, preferably in water, at 10°C-30°C, preferably 10-25°C, more preferably at 15°C-20°C, and mixing the two suspensions. The mixing can be performed at room temperature, preferably at 10°C-20°C since the solution is less viscous and allows easier mixing at a lower temperature. The remaining ingredients, such as an API, preferably hydrophilic API, filler, binder, sweetener, flavor, and/or permeation enhancer are added and mixed until the solution is substantially homogeneous. The aqueous solution, suspension, or dispersion is then poured into containers, molds shaped into a variety form, or preformed blisters (PVC or PVC/PVDC or Aclar and aluminum foil or AUAL). These are then loaded into a lyophilizer wherein the materials are first frozen and then lyophilized. Lyophilization is performed as described Lafon, U.S. Patent No. 4,616,047, Nguyen et al., U.S. Patent No. 5,843,347, and Blonde et al., U.S. Patent No. 3,855,712, all of which are herein incorporated by reference. Typically, freezing of the preparation is performed at very low temperature, i.e., -30°C to -45°C. The molecules of all the ingredients are immobilized and their properties remain unaltered as the rate of chemical reactions is nearly zero at this low temperature. Then the frozen preparations are lyophilized at low temperature and under low pressure where ice is directly converted into vapor phase. The lyophilization process is performed under a pressure within the range of about 5 to 500 μbar (4 X $10^{-2}$ mmHg to about 4 X $10^{-1}$ mmHg.) The secondary drying is operated at a temperature of about 20-60°C and a pressure within the range of about 20-50 μbar.

In another preferred embodiment, a lyophilized mucoadhesive layer (b) of the present invention containing a hydrophobic and/or lipophilic API(s) is prepared by preparing an oil-in-water emulsion. This is carried out by first preparing an oil suspension or solution containing a lipophilic API(s) and other lipophilic compounds, such as emulsifying agents, oils, flavoring agents, coloring agent and/or permeation enhancer. Then an oil-in-water emulsion is prepared by any process known to obtain an oil-in-water emulsion. The aqueous phase used to prepare the oil-in-water emulsion can contain all or part of the hydrophilic compounds necessary to obtain the dosage form, such as gelling agents, bioadhesive polymer, fillers, binders, sweeteners, coloring agents, buffers, and/or permeation enhancers. These two suspensions are then mixed until it is substantially homogeneous, poured into containers, molds shaped into a variety form, or preformed blisters and lyophilized.

Unlike the prior art, the methods of making a lyophilized mucoadhesive layer (b) of the present invention preferably do not comprise any step of breaking, cutting, milling or pulverizing of the lyophilized oral solid dosage forms and/or applying compressive force to form a compressed oral solid dosage form. Because the lyophilized mucoadhesive layer (b) of the present invention may be made without using any compressive force, it allows for faster dissolution, optimized bioavailability, rapid onset of action, uniform distribution of the API(s) when present and improvement in stability. It also provides advantages in terms of the properties of the resulting dosage form including density, porosity and/or hardness. In addition, the method of the present invention does not include any step of granulation (i.e., wet or dry granulation), simplifying the manufacturing process.

Once lyophilized, it is contemplated that the dosage forms may be stamped, painted, coated, printed, etc. These dosage forms may be stored in bulk, in blister packs, in conventional openable and reclosable multi-tablet bottles or other similar packaging. The preferred packaging is to store them in sealed blister packs.

The following examples are provided as non-limiting illustrations of the present invention.

EXPERIMENTAL PART

*Example 1: Lyophilized dosage layer (a)/lozenge laver (b)*

Modafinil bilayer lyophilized dosage form: taste-masked formulation

Layer (a): lyophilized formulation

| Modafinil | 82.85% |
|---|---|
| Sucralose | 5.85% |
| Methylcellulose | 0.58% |
| Flavor | 9.75% |
| $Na_2HPO_4$ | 0.98% |
| Purified water disappearing after lyophilization | QS |

Layer (b): lozenge

| Hypromellose | 20% |
|---|---|
| Lactose | 60% |
| Povidone K30 | 12.5% |

(continued)

| Sucralose | 2% |
|---|---|
| Flavor | 5% |
| Magnesium stearate | 0.5% |

Preparation of layer (a): prepare water suspension composed of water, modafinil, sucralose, methylcellulose, dibasic phosphate, flavour and mix until obtaining homogenised and stable suspension able to be filled by means of syringe or volumetric pumps.

Preparation of lozenge (b): mix approximately 10-15 minutes hypromellose, lactose, povidone, sucralose, flavour using a powder mixer (i.e.Turbula mixer), add magnesium stearate and mix again for 1-2 minutes. Transfer the mixed powder to the tablet press and compress with moderate compression force (< 1t) using flat punches. Lozenges attributes: diameter 13-16 mm, thickness 1-2 mm, weight 100-200 mg, hardness: 10-20N, disintegration time 30-40 minutes.

Preparation of the corresponding bilayer dosage form: distribute into preformed blister cards of 6 to 10 cavities of diameter 13-17 mm and height 5-6 mm: one lozenge (b) per cavity, cool the blisters at a temperature of 0°C and add the suspension (a) in order to have 85 mg, 100 mg, 170 mg, 200 mg, 255 mg of modafinil. Freeze the blisters' contents at a temperature below -25°C and freeze-dry with a pressure comprised between 250-350 μbar, followed by a secondary drying at a temperature 30-50°C and pressure below 50 μbar during 60-120 minutes.

Attributes of the bilayer dosage form:

| Modafinil | 100 mg | 200 mg |
|---|---|---|
| Weight | 270 mg | 390 mg |
| Hardness | 40-60 N | 80-100 N |
| Disintegration time, lyophilized dosage form portion | 10 seconds | 15 seconds |
| Disintegration time, lozenge portion | 30 minutes | 40 minutes |
| Bitterness | Masked | masked |

*Examples 2-3-4-5: Lyophilized layer (a)/lozenge (b)*

Modafinil bilayer lyophilized dosage form IR/SR (combination of immediate release (IR) and sustained release (SR))

Layer (2a)-(3a)-(4a)-(5a): IR lyophilized formulation

| Modafinil | 82.85% |
|---|---|
| Sucralose | 5.85% |
| Methylcellulose | 0.58% |
| Flavor | 9.75% |
| $Na_2HPO_4$ | 0.98% |
| Purified water disappearing after lyophilization | QS |

Layer (2b)-(3b)-(4b)-(5b): SR tablet

|  | (2b) | (3b) | (4b) | (5b) |
|---|---|---|---|---|
| Modafinil granules* (92%) | 50% | 50% | 50% | 50% |
| Hypromellose** | 30% | 20% | 10% | 0% |
| Lactose DC | 10% | 20% | 30% | 40% |
| Povidone(K30) | 6% | 6% | 6% | 6% |
| Sucralose | 1% | 1% | 1% | 1% |
| Flavor | 2.5% | 2.5% | 2.5% | 2.5% |
| Magnesium stearate | 0.5% | 0.5% | 0.5% | 0.5% |
| *Modafinil granules with Eudragit® E100, potency 89-95%<br>** different grade of Methocel®: K4M, 100, 4000, 15000, 100000 cps<br>Manufacturing process according to example 1.<br>The formulations 2-3-4-5 show different dissolution profiles. | | | | |

Figure 3 shows the dissolution profiles of examples 4 and 5. In these two formulations, 50% of the drug is contained in the immediate release portion and 50% is contained in the sustained release portion of the bilayer lyophilized dosage form. The release profile of the sustained release portion depends on the range of hydrophilic matrix.

*Example* 6: *Lyophilized laver (a)/lyophilized mucoadhesive layer (b)*

Layer (a): Lyophilized layer (a)

| Modafinil | 82.85% |
|---|---|
| Sucralose | 5.85% |
| Methylcellulose | 0.58% |
| Flavor | 9.75% |
| $Na_2HPO_4$ | 0.98% |
| Purified water disappearing after lyophilization | QS |

Layer (b): lyophilized mucoadhesive layer (b)

| Poloxamer 407 | 10% |
|---|---|
| Methylcellulose | 3.33% |
| Dextran70 | 3.33% |
| Sucralose | 3.33% |
| Flavor | 13.33% |
| Mannitol | 66.66% |
| Purified water disappearing after lyophilization | QS |

Preparation of lyophilized mucoadhesive layer (b) by stirring at a temperature approximately 60°C the methyl cellulose and at a temperature around 10°C the poloxamer 407 and mixing at room temperature the two solutions of polymers, introduce in the polymers solution dextran 70, sucralose, flavour and mannitol and mixed until obtaining a homogeneous suspension.

Preparation of layer (a) according to example 1

Preparation of the bilayer dosage form by distributing into blister cards of 6-10 cavities the suspension (b), cool this suspension at -10°C and add the suspension (a) and freeze the 2 layers of suspension at -30°C/-40°C then freeze-dry the product.

Drug product attributes:

| Modafinil | 100 mg |
|---|---|
| Unit weight | 270 mg |
| Disintegration time | 50-60 seconds |
| Hardness | 20-30N |

*Example 7: Lyophilized layer (a)/Lozenge (b)*

Ondansetron bilayer lyophilized dosage form: taste-masked formulation

Layer (a): Lyophilized layer

| Ondansetron HCl | 3.33% |
|---|---|
| Sucralose | 3.33% |
| Flavor | 10.00% |
| Dextran 70 | 6.67% |
| Mannitol | 76.67% |
| Purified water disappearing after lyophilization | QS |

Layer (b): Lozenge

| Hypromellose | 5% |
|---|---|
| Sucralose | 5% |
| Flavor | 5% |
| Povidone K30 | 20 % |
| Lactose | 64% |
| Magnesium stearate | 1% |

Preparation of layer (a), layer (b) and the bilayer dosage form according to example 1.

Drug product attributes:

| Ondansetron HCl | 5 mg |
|---|---|
| Unit weight | 250 mg |
| Disintegration time - Lyophilized dosage form portion | 5-10 seconds |
| Disintegration time - | 10-15 minutes |
| Hardness | 40 N |
| Bitterness | masked |

*Example 8: Lyophilized layer (a)/ lyophilized mucoadhesive layer (b)*

The obtained bilayer is a taste-masked formulation.

Layer (a): formulation containing Ondansetron similar to layer (a) of example 7.

Layer (b) is a lyophilized mucoadhesive layer suitable for taste masking.

Layer (b): lyophilized mucoadhesive layer formulation

| Poloxamer 407 | 8.8% |
|---|---|
| Methylcellulose | 6.3% |
| Dextran70 | 12.5% |
| Sucralose | 2.0% |
| Neotame | 0.2% |
| Isomalt | 10.0% |
| Flavor | 10.0% |
| Mannitol | 54.9% |
| Purified water disappearing after lyophilization | QS |

Preparation of layer (a) according to example 1; of layer (b), and bilayer dosage form according to example 6.

Drug product attributes:

| Ondansetron HCl | 5 mg |
|---|---|
| Unit weight | 230 mg |
| Disintegration time - Lyophilized dosage form (a) | 20-30 seconds |
| Hardness | 30 N |
| Bitterness | masked |

*Example 9: Lyophilized layer (a)/ Lozenge (b)*

Layer (a) is for immediate release, lozenge (b) is for extended release.

Lyophilized layer (a):

EP 1 980 245 A1

| Tramadol HCl | 50 mg |
|---|---|
| Sucralose | 5 mg |
| Neotame | 0.5 mg |
| Methylcellulose | 2.5 mg |
| Dextran 70 | 7 mg |
| Mannitol | 135 mg |
| Purified water disappearing after lyophization | QS |

Layer (b): Lozenge

| Tramadol HCl | 50 mg | 100 mg | 150 mg |
|---|---|---|---|
| Hypromellose (Methocel® K4M) | 10 mg | 20 mg | 30 mg |
| Sucralose | 2 mg | 4 mg | 6 mg |
| Neotame® | 0.4 mg | 0.8 mg | 1.2 mg |
| Povidone K30 | 6 mg | 12 mg | 18 mg |
| Lactose | 41.1 mg | 82.2 mg | 12.3 mg |
| Magnesium stearate | 0.5 mg | 1.0 mg | 1.5 mg |

Preparation of layer (a), layer (b) and bilayer dosage form performed according to a process similar to example 1, except tramadol being added with ingredients in first step similar to (a).

Drug product attributes:

| Tramadol HCl | 100 mg | 150 mg | 200 mg |
|---|---|---|---|
| Unit weight | 310 mg | 420 mg | 530 mg |

## Claims

1.  A bilayer dosage form comprising:

    - an upper layer (a) comprising a lyophilized dosage form of active pharmaceutical ingredient(s) (API(s)), and
    - a base line layer (b) formulated to adhere to the oral mucosa and intended for oral and/or transmucosal absorption, and for delayed, sustained or extended release of API(s) and/or excipient(s).

2.  The bilayer dosage form according to claim 1, wherein layer (b) is chosen from the group consisting in tablets, lozenges or lyophilized mucoadhesive dosage forms.

3.  The bilayer dosage form according to anyone of the preceding claims, wherein lyophilized layer (a) comprises further ingredients chosen from fillers, binders, surfactants, cosurfactants, excipients, lipid components.

4.  The bilayer dosage form according to claim 1 or 3, wherein excipients comprise flavour(s) and sweetener(s).

5.  The bilayer dosage form according to anyone of the preceding claims, wherein lyophilized layer (a) comprises:

19

| - one or more hydrophilic API(s) | 0.001-88 % |
|---|---|
| - one or more filler(s) | 5-90 % |
| - one or more binder(s) | 2-20 % |
| and optionally - one or more surfactant(s) | 0-5 % |
| - one or more flavour(s) or sweetener(s) | 0-15 % |
| - purified water disappearing after lyphilization | QS |

6. The bilayer dosage form according to anyone of claims 1 to 4, wherein lyophilized layer (a) comprises:

| - one or more lipohilic API(s) | 0.001-70% |
|---|---|
| - one or more lipid component(s) | 5-50% |
| - one or more surfactants) or cosurfactant(s) | 1-20% |
| - one or more binder(s) or filler(s) | 10-90% |
| and optionally - one or more flavour(s) or sweetener(s); | 0-15% |
| - purified water disappearing after lyphilization | QS |

7. The bilayer dosage form according to anyone of the preceding claims, wherein layer (b) comprises one or more bioadhesive polymer(s).

8. The bilayer dosage form according to anyone of the preceding claims, wherein layer (b) is a lyophilized mucoadhesive dosage form.

9. The bilayer dosage form according to claim 8, wherein the lyophilized mucoadhesive layer (b) comprises:

- a gelling agent, and
- a bioadhesive polymer,

wherein said lyophilized mucoadhesive layer (b) adheres to an oral mucosal tissue for a time sufficient to release said API(s) through said oral mucosal tissue.

10. The bilayer dosage form according to claim 9, wherein said lyophilized mucoadhesive layer (b) adheres to the oral mucosal tissue for about 10 minutes to about 180 minutes.

11. The bilayer dosage form according to claim 9 or 10, wherein said gelling agent is selected from the group consisting in a polysaccharide, guar gum, arabic gum, tragacanth gum, xanthan gum, carraghenan, pectin, starch, poloxamer 188, poloxamer 407, polaxamine, acrylic polymer, carbopol and a mixture thereof.

12. The bilayer dosage form according to claim 9 or 11, wherein said gelling agent is present in an amount from of about 0.5% to about 50% relative to the total weight of the dosage form.

13. The bilayer dosage form according to anyone of claims 8 to 12, wherein said bioadhesive polymer is selected from the group consisting in a cellulose derivative, cellulose, carboxymethyl cellulose, sodium carboxymethyl cellulose, methyl cellulose, hydroxypropyl cellulose, hydroxyl propyl methyl cellulose, hydroxyl ethyl cellulose and a mixture thereof.

14. The bilayer dosage form according to claim 13, wherein said bioadhesive polymer is present in an amount of from about 0.5% to about 30% relative to the total weight of the dosage form.

15. The bilayer dosage form according to anyone of claims 8 to 14, wherein said lyophilized mucoadhesive layer (b) comprises a water insoluble and/or lipophilic API(s) and further comprises an oil and an emulsifying agent.

16. The bilayer dosage form according to claim 15, wherein said oil is selected from the group consisting in Miglyol® 810-812-818, mono-triglyceride ester, di-triglycerides ester, fatty acid of glycerol, mineral oil, vegetable oil, a reformed vegetable oil and a mixture thereof.

17. The bilayer dosage form according to claim 15, wherein said emulsifying agent is selected from the group consisting in polysorbate 20, polysorbate 60, polysorbate 80, sucroester 7, sucroester 11, sucroester 15, sorbitan, poloxamer, dioctyl sulfosuccinate, polyethoxyethers of fatty glycerides, lecithins and a mixture thereof.

18. The bilayer dosage form according to anyone of claims 8 to 17, wherein said lyophilized mucoadhesive layer (b) further comprises excipient(s) and/or functional excipient(s) for lyophilized dosage forms.

19. The bilayer dosage form according to claim 18, wherein said excipient is selected from the group consisting in a binder, filler, permeation enhancer, defoaming agent, sweetener, coloring agent, flavoring agent and a mixture thereof.

20. The bilayer dosage form according to claim 19, wherein said binder is selected from the group consisting in Dextran 70, povidone 12, povidone 17, povidone 30, copovidone, gelatin, starch, pregelatinized starch and a mixture thereof.

21. The bilayer dosage form according to claim 19, wherein said filler is selected from the group consisting in mannitol, dextrose, sorbitol, isomalt, glycocolle, cyclodextrin derivative, cyclodextrin, glucose, maltodextrine, lactose, sucrose, calcium carbonate and a mixture thereof.

22. The bilayer dosage form according to claim 19, wherein said permeation enhancer is selected from the group consisting in sodium salicylate, citric acid, sodium acid carbonate, sodium glyococholate, sodium taurocholate, sodium carbonate, sodium bicarbonate or a mixture thereof.

23. The bilayer dosage form according to claim 19, wherein said buffer is selected from the group consisting in monobasic potassium phosphate, anhydrous dibasic sodium phosphate, sulfuric acid, sodium hydroxide, citric acid, sodium carbonate, sodium bicarbonate and a mixture thereof.

24. The bilayer dosage form according to claim 8, wherein the lyophilized mucoadhesive layer (b) comprises:

a gelling agent, and
a bioadhesive polymer;

wherein said lyophilized mucoadhesive layer (b) exhibits at least one of the following characteristics: the density of said lyophilized mucoadhesive layer (b) is from about 100 mg/ml to about 900 mg/ml, the porosity of said lyophilized mucoadhesive layer (b) is from about 10% to about 90%, and the hardness of said lyophilized mucoadhesive layer (b) is from about 4.5N to about 100N.

25. The bilayer dosage form according to claim 24, wherein said lyophilized mucoadhesive layer (b) exhibits at least two of the following characteristics: a density is from about 100 mg/ml to about 900 mg/ml, a porosity is from about 10% to about 90%, and a hardness is from about 4.5N to about 100N.

26. The bilayer dosage form according to claim 24 or 25, wherein said density ranges from about 300 mg/ml to about 600 mg/ml.

27. The bilayer dosage form according to claim 24, 25 or 26, wherein said porosity ranges from about 30% to about 60%.

28. The bilayer dosage form according to anyone of claims 24 to 27, wherein said hardness ranges from about 10N to about 100N.

29. The bilayer dosage form according to anyone of the preceding claims, wherein layer (b) is a placebo composition.

30. The bilayer dosage form according to claim 29, wherein said placebo composition comprises flavouring or sweetening system.

31. The bilayer dosage form according to anyone of the preceding claims, wherein layer (b) comprises an identification mark.

32. The bilayer dosage form according to anyone of the claims 8 to 31, wherein said lyophilized mucoadhesive layer (b) comprises:

| | |
|---|---|
| - one or more gelling agent(s) | 5-20% |
| - one or more bioadhesive polymer(s) | 5-20% |
| - one or more binder(s) or filler(s) | 20-90% |
| And optionally :<br>- one or more API(s)<br>- one or more surfactant(s)<br>- one or more flavour(s) or sweetener(s)<br>- one or more permeation enhancers | 0-50%<br>0-5%<br>0-15%<br>0-10% |
| - purified water disappearing after lyphilization | QS |

33. The bilayer dosage form according to claim 1 or 7, wherein layer (b) is a tablet or a lozenge.

34. The bilayer dosage form according to claim 33, wherein said tablet or lozenge comprises:

| | |
|---|---|
| - one or more filler(s) or binder(s) | 5-80% |
| - lubricant | 0.1-2% |
| - one or more bioadhesive polymer | 1-95% |
| and optionally<br>- one or more API(s)<br>- one or more flavour(s) or sweetener(s)<br>- one or more hydrophilic matrix<br>- one or more buffer(s) | 0-50%<br>0-20%<br>0-95%<br>0-10% |

35. The bilayer dosage form according to anyone of the preceding claims, wherein said bilayer dosage form has a maximum diameter of from about 5 to about 20 mm and a thickness of from about 2 to about 8 mm.

36. The bilayer dosage form according to anyone of the preceding claims, wherein said layer (b) comprises API(s).

37. The bilayer dosage form according to anyone of the preceding claims, wherein said API(s) comprises:

(i) non-coated API(s), and
(ii) coated API(s).

38. The bilayer dosage form according to anyone of the preceding claims, wherein said API(s) is selected from the group consisting in fentanyl, modafinil, ondansetron, granisetron, sumatriptan, tramadol, desmopressin, calcitonin, insulin, GLP-1, PPY, oxycontin, or taxol and a mixture thereof.

39. The bilayer dosage form according to anyone of the preceding claims, wherein said API(s) is present in an amount of from about 0.001% to about 70% relative to the total weight of the dosage form.

40. A process of preparing a bilayer dosage form according to anyone of the preceding claims comprising the steps of:

i. providing a suspension suitable for preparing the lyophilized layer (a);
ii. providing layer (b) in the form of a tablet, lozenge or a suspension suitable for preparing the lyophilized mucoadhesive layer (b);
iii. cooling layer (b) at a temperature comprised between -20°C/+10°C;
iv. adding said suspension suitable for preparing the lyophilized layer (a) on said layer (b) in the form of a tablet, lozenge or a suspension suitable for preparing the lyophilized mucoadhesive layer (b);
iv. freezing the bilayer system obtained; and
v. lyophilizing.

41. A process of preparing a bilayer dosage form according to anyone of the preceding claims comprising the steps of:

    i. providing a suspension suitable for preparing the lyophilized layer (a) in a mould;
    ii. providing a suspension suitable for preparing the lyophilized mucoadhesive layer (b);
    iii. cooling layer (a) at a temperature comprised between -20°C/+10°C;
    iv. adding said suspension suitable for preparing the lyophilized mucoadhesive layer (b) on said suspension suitable for preparing the lyophilized layer (a);
    iv. freezing the bilayer system obtained; and
    v. lyophilizing.

42. The process step according to claim 40 or 41, wherein said suspension suitable for preparing the lyophilized mucoadhesive layer (b) is obtained by:

    (a) dissolving a gelling agent in water at a temperature below 25°C;
    (b) dissolving a bioadhesive polymer in water at a temperature below 100°C; and
    (c) mixing said suspensions from steps (a) and (b).

43. The process according to claim 42, which further comprises the step of:

    (d) adding API(s) to said suspension from step (c) and mixing until the suspension is substantially homogeneous.

44. The process according to claim 42 or 43, wherein said step (c) of mixing is performed at a temperature between about 10°C and about 30°C.

45. The method according to claims 42 to 44, wherein said step (c) of mixing is performed at a temperature between about 15°C and about 25°C.

46. The process according to claim 40 or 41, wherein said suspension suitable for preparing lyophilized mucoadhesive layer (b) is obtained by:

    (a) preparing an aqueous suspension containing at least one hydrophilic compound,
    (b) preparing an oil suspension containing at least one lipophilic compound,
    (c) mixing said suspension phase of step (a) and said oil suspension from step (b) to form an emulsion.

47. The method according to claim 46, wherein said hydrophilic compound is selected from a gelling agent, bioadhesive polymer, binder, filler and hydrophilic API(s).

48. The method according to claim 46 or 47, wherein said lipophilic compound is an emulsifying agent, sweetener, flavoring agent and lipophilic API(s).

49. The process according to anyone of claims 40 to 47, wherein the suspension suitable for preparing the lyophilized layer (a) is obtained by preparing a liquid preparation of the API(s) and optional further ingredients as defined in claim 3.

50. The process according to anyone of claims 40 to 49, wherein said layer (b) in the form of a tablet, lozenge, or a suspension suitable for preparing the lyophilized mucoadhesive layer (b) or said suspension suitable for preparing said lyophilized layer (a) is distributed into a mould prior to adding the second component.

51. The process according to anyone of claims 40 to 50, wherein layer (b) in the form of a tablet or a lozenge is prepared by direct compression or compaction.

**52.** The process according to claim 50, wherein it further comprises the step of sealing the mould containing the bilayer dosage form.

**53.** The process according to anyone of claims 40 to 52, wherein the freezing step is carried out at temperature comprised between -45°C and -15°C.

**54.** The process according to anyone of claims 40 to 53, wherein the freeze-drying step is carried out at a temperature comprised between -25°C and 25°C under pressure comprised between 10 and 100 microbars during 60 to 120 minutes.

**55.** Use of a bilayer dosage form according to anyone of claims 1 to 39, containing a therapeutically effective amount of at least one API for the preparation of a medicament for treating a patient, said medicament being suitable for:

(a) placing said bilayer dosage form onto an oral mucosal tissue of a patient in need of treatment; and
(b) allowing said bilayer dosage form to release said therapeutically effective amount of said at least one API through said oral mucosal tissue while adhering to said oral mucosal tissue.

Layer (a)
Layer (b)

**Figure 1**

2a

Lyophilized layer: fast melt, bitter API

Placebo lyophilized mucoadhesive layer (bioadhesive + flavor + sweetener)

**Figure 2a**

2b

Lyophilized layer: fast melt, bitter API

Placebo tablet (bioadhesive + flavor + sweetener)

**Figure 2b**

2c

Lyophilized layer: fast melt, fast absorbed API

Lyophilized mucoadhesive layer: API bioadhesive sustained release or local action

**Figure 2c**

2d

Lyophilized layer: API fast melt

Tablet API + bioadhesive + sustained release or local action

**Figure 2d**

2e

Lyophilized layer: API fast melt

Tablet + bioadhesive with identification

**Figure 2e**

**Figure 3**

**European Patent Office**

**EUROPEAN SEARCH REPORT**

Application Number

EP 07 29 0444

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 00/59423 A (WATSON PHARMACEUTICALS INC [US]) 12 October 2000 (2000-10-12)<br>* examples 2,3,5 *<br>* claim 1 *<br>----- | 1-55 | INV.<br>A61K9/24<br>A61K31/165<br>A61K31/135 |
| Y | WO 01/37814 A1 (ROBERT GORDON UNIVERSITY [GB]; PARK CALUM [GB]; MUNDAY DALE [GB]; BAIN) 31 May 2001 (2001-05-31)<br>* figure 1 *<br>* claim 1 *<br>----- | 1-55 | |
| Y | WO 03/063840 A (SANTARUS INC [US]; WIDDER KEN [US]; HALL WARREN [US]; OLMSTEAD KAY [US]) 7 August 2003 (2003-08-07)<br>* page 17, line 5 - page 18, line 27 *<br>* example 3 *<br>* figures 2,3 *<br>* page 16, line 15 - line 19 *<br>----- | 1-55 | |
| Y | UZUNOGLU B ET AL: "FORMULATION OF A BIOADHESIVE BILAYERED BUCCAL TABLET OF NATAMYCIN FOR ORAL CANDIDIASIS" PROCEEDINGS OF THE INTERNATIONAL SYMPOSIUM ON CONTROLLED RELEASE BIOACTIVE MATERIALS, XX, XX,<br>vol. 27, 2000, pages 1186-1187,<br>XP008056535<br>ISSN: 1022-0178<br>* page 1186, column 1, last paragraph - column 2, paragraph 1 *<br>----- | 1-55 | TECHNICAL FIELDS SEARCHED (IPC)<br><br>A61K |

-/--

The present search report has been drawn up for all claims

| Place of search | Date of completion of the search | Examiner |
|---|---|---|
| Munich | 20 September 2007 | Sindel, Ulrike |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.............................................................

& : member of the same patent family, corresponding document

| | **European Patent Office** | **EUROPEAN SEARCH REPORT** | **Application Number** EP 07 29 0444 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | PARK C R ET AL: "Development and evaluation of a biphasic buccal adhesive tablet for nicotine replacement therapy" INTERNATIONAL JOURNAL OF PHARMACEUTICS, AMSTERDAM, NL, vol. 237, no. 1-2, 26 April 2002 (2002-04-26), pages 215-226, XP002279251 ISSN: 0378-5173 * abstract * * figure 1 * * paragraph [02.3] * | 1-55 | |
| A | REMUNAN-LOPEZ C ET AL: "Design and evaluation of chitosan/ethylcellulose mucoadhesive bilayered devices for buccal drug delivery" JOURNAL OF CONTROLLED RELEASE, ELSEVIER, AMSTERDAM, NL, vol. 55, no. 2-3, 13 November 1998 (1998-11-13), pages 143-152, XP004143509 ISSN: 0168-3659 * abstract * | 1-55 | |
| Y | EP 1 621 186 A (CEPHALON FRANCE [FR]) 1 February 2006 (2006-02-01) * paragraph [0033] * * paragraph [0149] - paragraph [0154] * | 1-55 | TECHNICAL FIELDS SEARCHED (IPC) |
| A | US 5 384 124 A (COURTEILLE FREDERIC [FR] ET AL) 24 January 1995 (1995-01-24) * column 6, line 56 - line 63 * * example 1 * * claim 1 * | 1-55 | |

-/--

The present search report has been drawn up for all claims

| Place of search | Date of completion of the search | Examiner |
|---|---|---|
| Munich | 20 September 2007 | Sindel, Ulrike |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent
Office

**EUROPEAN SEARCH REPORT**

Application Number

EP 07 29 0444

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2005/084530 A1 (RAO PAVULURI V [IN] ET AL) 21 April 2005 (2005-04-21)<br>* paragraph [0023] - paragraph [0024] *<br>* claims 11,25 *<br>----- | 1-55 | |
| | | | **TECHNICAL FIELDS SEARCHED (IPC)** |

The present search report has been drawn up for all claims

| Place of search | Date of completion of the search | Examiner |
|---|---|---|
| Munich | 20 September 2007 | Sindel, Ulrike |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

                

& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 07 29 0444

This annex lists the patent family members relating to the patent documents cited in the above-mentioned European search report.
The members are as contained in the European Patent Office EDP file on
The European Patent Office is in no way liable for these particulars which are merely given for the purpose of information.

20-09-2007

| Patent document cited in search report | | Publication date | Patent family member(s) | | Publication date |
|---|---|---|---|---|---|
| WO 0059423 | A | 12-10-2000 | AU | 764912 B2 | 04-09-2003 |
| | | | AU | 4178600 A | 23-10-2000 |
| | | | CA | 2333156 A1 | 12-10-2000 |
| | | | EP | 1089686 A1 | 11-04-2001 |
| | | | US | 6210699 B1 | 03-04-2001 |
| | | | US | 2001051186 A1 | 13-12-2001 |
| WO 0137814 | A1 | 31-05-2001 | AU | 1713501 A | 04-06-2001 |
| | | | EP | 1231900 A1 | 21-08-2002 |
| WO 03063840 | A | 07-08-2003 | CA | 2472103 A1 | 07-08-2003 |
| | | | EP | 1469839 A2 | 27-10-2004 |
| | | | JP | 2005521662 T | 21-07-2005 |
| | | | MX | PA04007169 A | 29-10-2004 |
| EP 1621186 | A | 01-02-2006 | AR | 050363 A1 | 18-10-2006 |
| US 5384124 | A | 24-01-1995 | NONE | | |
| US 2005084530 | A1 | 21-04-2005 | NONE | | |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6200604 B
- US 6974590 B
- US 4616047 A
- US 5843347 A, Nguyen
- US 3855712 A, Blonde
- US 4490407 A
- US 3939260 A
- US 6024981 A
- US 4949588 A
- US 5178878 A
- US 5055306 A

**Non-patent literature cited in the description**

- **SHIN et al.** Mucoadhesive and Physicochemical Characterization of Carbopol-Poloxamer Gels Containing Triamcinolone Acetonide. *Drug Development and Industrial Pharmacy,* 2000, vol. 26 (3), 307-312
- **CAFFAGGI et al.** Preparation and Evaluation of a Chitosan Salt-Poloxamer 407 Based Matrix for Buccal Drug Delivery. *Journal of Controlled Release,* 2005, vol. 102, 159-169
- **LIEBERMAN.** Pharmaceutical Dosage Form: Tablets. 1989, vol. 1, 732-36